# FASCICULE DE BREVET EUROPEEN

(11) **EP 0 842 411 B1**
(45) Date de publication et mention de la délivrance du brevet: **22.05.2002**
(21) Numéro de dépôt: 96927122.0
(22) Date de dépôt: 01.08.1996
(51) Int. Cl.: G01N 1/31

(54) **APPAREILLAGE D'ALIGNEMENT PARALLELE DE MACROMOLECULES ET UTILISATION**
VORRICHTUNG ZUR PARALLEL-AUSRICHTUNG VON MAKROMOLEKÜLEN UND VERWENDUNG DAVON
APPARATUS FOR THE PARALLEL ALIGNMENT OF MACROMOLECULES, AND USE THEREOF

(30) Priorité: 03.08.1995 FR 9509466
(43) Date de publication de la demande: 20.05.1998
(73) Titulaire: INSTITUT PASTEUR, 75015 Paris (FR)
(72) Inventeur: BENSIMON, Aaron, F-92160 Antony (FR); BENSIMON, David, F-75013 Paris (FR)
(74) Mandataire: Warcoin, Jacques
(86) Numéro de dépôt international: FR9601218
(87) Numéro de publication internationale: WO9706420

(56) Documents cités:
- EP-A- 0 180 792
- EP-A- 0 323 130
- FR-A- 2 109 233
- US-A- 3 165 108
- NUCLEIC ACIDS RESEARCH, vol. 22, no. 3, 1994, OXFORD GB, pages 492-497, XP002018119 R.M. ZIMMERMANN ET AL.: "DNA stretching on functionalized gold surfaces" cité dans la demande

## Description

La présente invention concerne un appareillage d'alignement parallèle de macromolécules sur un support solide par passage d'un ménisque.

On a décrit dans la demande de brevet en France n° 94 07444 un procédé d'alignement de macromolécules encore appelé "peignage moléculaire".

Contrôler la conformation de macromolécules représente un enjeu industriel important, par exemple dans la fabrication de capteurs ou d'assemblages moléculaires contrôlés ou encore dans les problèmes de détection et d'analyse. Il peut être intéressant d'avoir une conformation moléculaire allongée. A titre d'exemple, dans le cas où des polymères sont greffés sur un substrat, il a été proposé de les étendre par l'action d'un champ électrique, d'un écoulement ou à l'aide de pinces optiques. En particulier, en biologie, l'alignement de l'ADN - par électrophorèse (Zimmerman et Cox Nucl. Acid Res. 22, p 492, 1994), écoulement libre (Parra et Windle, Nature Genetics, 5, p 17, 1993 et WO 93/22463) ou dans un gel (Schwartz et al. Science 262, p 110, 1993 et USP 33531) ou à l'aide de pinces optiques (Perkins et al., Science 264 p 819, 1994 et aussi USP 5079169) - ouvre de nombreuses possibilités en cartographie, ou dans la détection de pathogènes.

Ces méthodes ne permettent en général qu'un alignement imparfait, ou encore transitoire - c'est-à-dire qu'on a relaxation de la molécule, une fois la contrainte disparue. Dans le cas des pinces optiques, la méthode est lourde, limitée à une seule molécule à la fois, et délicate à mettre en oeuvre par des personnels non qualifiés.

Il a été proposé (I. Parra et B. Windle et WO 93/22463) une technique particulière d'alignement de l'ADN par écoulement après lyse de cellule, puis séchage. L'alignement obtenu est très imparfait et inhomogène et de nombreux amas non alignés sont observés.

Dans la demande de brevet FR 94 07444, on a décrit une méthode originale et simple pour aligner des macromolécules sur la surface S d'un support caractérisée en ce que l'on fait se déplacer sur ladite surface S la ligne triple S/A/B (ménisque) résultant du contact d'un solvant A avec la surface S et un milieu B, lesdites macromolécules ayant une partie, notamment une extrémité, ancrée sur la surface S, l'autre partie, notamment l'autre extrémité, étant en solution dans le solvant A.

On a observé que le seul passage d'un ménisque sur des molécules dont une partie est ancrée sur un substrat, le reste de la molécule étant librement en solution permet de les aligner uniformément perpendiculairement au ménisque en mouvement les laissant adsorbées sur la surface derrière le ménisque. Ce phénomène est appelé ici "peignage moléculaire".

Plus précisément, l'étirement de la partie libre de la molécule se fait par le passage de la ligne triple S/A/B, constituant le ménisque entre la surface S, le solvant A et un milieu B qui peut être un gaz (en général de l'air) ou un autre solvant.

Dans un mode de réalisation particulier, le ménisque est un ménisque eau-air, c'est-à-dire que le solvant A est une solution aqueuse et le milieu B est de l'air.

Le déplacement du ménisque peut se faire en particulier par évaporation progressive du solvant A ou par voie mécanique par translation de la surface S.

Pour ce faire dans la demande de brevet FR 94 07444, une goutte de solvant contenant les molécules à aligner est placée entre deux supports dont un au moins correspond audit support de surface S et le ménisque est déplacé par exemple soit par évaporation, soit en déplaçant les deux supports l'un par rapport à l'autre.

On entend par "support", tout substrat dont la cohésion est suffisante pour résister au passage du ménisque.

Le support peut être constitué au moins en surface, par un polymère organique ou inorganique, un métal notamment de l'or, un oxyde ou sulfure de métal, un élément semi-conducteur ou un oxyde d'élément semi-conducteur, tel qu'un oxyde de silicium ou une combinaison de ceux-ci, telle que du verre ou une céramique.

On cite plus particulièrement le verre, le silicium oxydé en surface, le graphite, le mica et le sulfure de molybdène.

A titre de "support", on peut utiliser un support unique tel qu'une lame, des billes, notamment de polymère, mais aussi des formes quelconques telles que barre, fibre ou support structuré, et également des particules, qu'il s'agisse de poudres, notamment de poudres de silice, lesquelles peuvent d'ailleurs être rendues magnétiques fluorescentes ou colorées comme cela est connu dans les différentes technologies de dosage.

Le support est avantageusement sous forme de plaques.

Des macromolécules, telles que polymères quelconques, ou polymères biologiques tels que ADN, ARN ou Protéines, peuvent être ancrées par des méthodes quelconques sur un support.

La macromolécule à aligner peut être choisie parmi les macromolécules biologiques telles que les proteines, notamment les anticorps, antigènes, ligands ou leurs récepteurs, les acides nucléiques, ADN, ARN ou PNA, les lipides, les polysaccharides et leurs dérivés.

On a observé que la force d'étirement agit localement au voisinage immédiat du ménisque. Elle est indépendante de la longueur de la molécule, du nombre de molécules ancrées, et dans une large gamme, de la vitesse du ménisque. Ces caractéristiques sont particulièrement intéressantes pour aligner les molécules de façon homogène et reproductible.

On peut ajouter des éléments tensio-actifs dans le solvant A, notamment l'eau, et/ou le milieu B, notamment l'air, qui viennent modifier les propriétés des interfaces. L'étirement peut en effet être contrôlé par l'addition de tensio-actifs, ou par un traitement de surface adéquat.

Une trop grande attraction surface-macromolécule (par exemple un niveau trop élevé d'adsorption) peut gêner l'alignement des molécules par le ménisque, celles-ci restant adsorbées à la surface dans un état pas nécessairement étiré. De préférence, la surface présente un faible taux d'adsorption de ladite macromolécule, de sorte que seules les molécules ancrées sont alignées, les autres étant entraînées par le ménisque.

Cependant, on peut jouer sur les différences d'adsorption entre une partie de la macromolécule notamment ses extrémités et ses autres parties (en particulier pour de longues molécules, telles l'ADN ou le collagène) pour ancrer par adsorption les molécules par une partie notamment leur(s) extrémité(s) seulement, le reste de la molécule étant librement en solution, sur une très grande variété de surfaces et les aligner par passage du ménisque comme décrit précédemment.

L'adsorption d'une macromolécule sur une surface peut se contrôler aisément à l'aide du pH ou de la teneur du milieu ionique du milieu ou d'une tension électrique appliquée sur la surface. On change ainsi les charges surfaciques et les interactions électrostatiques (répulsives ou attractives) entre la surface et la molécule, ce qui permet de passer d'un état d'adsorption complet de la molécule sur la surface à une absence totale d'adsorption. Entre ces deux cas extrêmes, il existe une plage des paramètres de contrôle où l'adsorption se fait préférentiellement par l'extrémité des molécules et que l'on utilisera donc, avec avantage, pour les ancrer à la surface, puis les aligner par le passage du ménisque.

Les molécules, une fois alignées, adhèrent fortement à la surface. Dans le cas de l'ADN, elles ont pu être observées par fluorescence, plusieurs mois après leur alignement.

La technique consiste donc dans un premier temps, en l'ancrage sur la surface prétraitée d'un support solide en solution de macromolécules, notamment d'ADN par leur(s) extrémité(s). Cet ancrage spécifique -seules les extrémités des molécules adhèrent à la surface du support- nécessite un contrôle précis de certains paramètres physico-chimiques de la solution : pH, température. Cette étape est désignée par la suite sous le nom d' *"étape d'incubation".* La durée d'incubation a une influence sur le taux d'ancrage par unité de surface.

Dans un deuxième temps, le système passe de la configuration : surface dans la solution, à une configuration : surface hors de la solution. Ce passage a d'abord été réalisé par évaporation naturelle d'une goutte de solution déposée sur la surface prétraitée. Lors de l'évaporation, le bord de la goutte, qui constitue un ménisque, c'est-à-dire une interface triple : surface-solution-air, se déplace du fait de la diminution du volume de la goutte. L'effet du passage du ménisque sur la surface est une force de traction sur les molécules d'ADN ancrées sur la surface, qui a pour conséquence leur étirement dans la direction de recul du ménisque, c'est-à-dire perpendiculairement à l'axe du ménisque. Les molécules étirées adhèrent de façon durable à la surface. Cette étape constitue le *peignage moléculaire* proprement dit.

L'appareillage selon la présente invention permet de réaliser cette seconde étape d'une manière différente. Au lieu d'utiliser le recul du ménisque d'une goutte d'eau, qui est approximativement centripète dans la direction du centre de la goutte, ce qui donne lieu à un alignement parallèle des molécules uniquement localement, on a recours à un ménisque dont le recul provoque un alignement parallèle des macromolécules sur toute leur longueur.

Plus précisément, la présente invention a pour objet un appareillage tel que défini dans la revendication 1.

Dans un mode de réalisation, la surface S peut être retirée de la solution ou inversement, la solution peut être retirée de la surface S, le ménisque étant en mouvement relatif.

Avantageusement, lesdits moyens d'immersion du support solide et lesdits moyens de déplacement rectiligne relatif du ménisque et de la surface S consistent en des moyens de translation verticale de ladite surface S à l'aplomb de la surface de ladite solution.

Dans un mode de réalisation, lesdits moyens de translation verticale de ladite surface S comprennent :
- des moyens de préhension du support solide et,
- des moyens de guidage et moteur pour provoquer la translation desdits moyens de préhension le long d'un axe vertical disposé à l'aplomb dudit récipient.

En particulier, lesdits moyens de translation verticale de la surface S comprennent :
- des moyens de préhension du support solide consistant en une tige verticale disposée à l'aplomb dudit récipient et supportant des moyens de pincement du support solide, et
- des moyens de guidage et moteur pour commander la translation verticale de la tige entre une position basse d'immersion et une position haute de retrait.

Avantageusement donc l'appareillage, selon la présente invention, comporte en outre, notamment lorsque le support solide est constitué par une lame:
- un premier compartiment de stockage des supports solides notamment des lames avant immersion dans ledit récipient, et
- un deuxième compartiment de stockage des supports solides notamment des lames sur lesquelles les macromolécules sont alignées après leur retrait dudit récipient.

Dans ce cas, il peut comporter en outre des moyens pour présenter successivement lesdites lames à l'aplomb dudit premier compartiment dudit récipient et dudit deuxième compartiment.

En particulier, lorsque lesdits compartiments et ledit récipient sont alignés, l'appareillage comporte en outre des moyens de guidage et des moyens moteur pour provoquer la translation horizontale de la tige quand elle est en position haute pour permettre de présenter lesdites lames à l'aplomb desdits compartiments et dudit récipient.

L'appareillage selon la présente invention comporte de nombreux avantages :
- le parfait parallélisme des molécules peignées sur toute leur longueur,
- la possibilité d'aligner des macromolécules de taille allant jusqu'à 1200 Kilobases alors que le procédé par évaporation de goutte ne permettait pas l'alignement de molécules de taille supérieure à 500 Kilobases,
- l'utilisation d'un volume de solution correspondant à la contenance du réservoir, permet l'incubation d'un nombre quasi illimité de lames (dans la limite de la stabilité chimique des molécules en solution). Il est en effet possible d'incuber séquentiellement un nombre quelconque de lames lorsque c'est la lame qui est retirée de la solution, comme c'était le cas dans le procédé reposant sur l'évaporation d'une goutte de solution,
- n'importe quelle taille de lame, et plus généralement, n'importe quel support prétraité pour permettre l'ancrage des molécules, peuvent être utilisés. Il suffit en effet d'adapter le volume du réservoir au support à incuber,
- les phases d'incubation et d'étirement étant dissociées, il est possible de varier le temps d'incubation et la vitesse d'étirement indépendamment, alors que ceux-ci sont plus difficilement contrôlables dans le procédé par évaporation de goutte,
- dans le cas d'un mode d'utilisation où la solution est retirée du réservoir, les lames restant fixes, on peut réaliser des incubations successives des lames dans le même réservoir, mais au sein de milieux différents.

L'appareillage de peignage moléculaire selon la présente invention, permet ainsi de passer du stade de la recherche fondamentale à ceux de la recherche appliquée ou même de l'instrumentation standard de laboratoire, en ce qui concerne toutes les applications possibles du peignage moléculaire (cartographie, diagnostic, etc...).

De façon appropriée, lesdits moyens de déplacement du ménisque permettent le déplacement du ménisque à une vitesse de 10 à 100 µm/sec.

La vitesse d'extraction des lames hors de la solution n'a aucune influence sur le taux d'étirement des molécules peignées, dans cette gamme de vitesse. Cette vitesse, qui peut sans doute être dépassée sans influer sur la qualité du peignage, permet de peigner une lame couvre-objet prétraitée de 22 x 22 mm² en moins de cinq minutes.

De façon appropriée, le volume dudit récipient est de 1 à 10 ml.

Le volume de solution dans le réservoir étant notablement plus grand que celui d'une goutte (quelques ml contre quelques dizaines de µl), il est possible d'en varier la température sans trop augmenter le taux d'évaporation : nous projetons d'étudier l'influence de ce paramètre sur le taux d'ancrage des molécule.

Le pH de la solution est crucial pour la spécificité de l'ancrage comme l'ont montré des études préliminaires. L'appareillage selon la présente invention, comporte avantageusement un système de contrôle de ce paramètre.

A ce titre, l'appareillage permet le peignage en série d'un grand nombre de lames prétraitées puisque la solution d'ADN contenue dans un réservoir de quelques ml permet d'en préparer un nombre quasi illimité.

Par "ancrage" de la macromolécule sur la surface il faut entendre une fixation résultant d'une réactivité chimique aussi bien par un lien covalent que par un lien non covalent tel qu'une liaison résultant d'interactions physico-chimiques, telle l'adsorption comme décrit ci-dessus.

Cet ancrage de la macromolécule peut se faire directement sur (ou avec) la surface, ou indirectement, c'est-à-dire par l'intermédiaire d'un lien tel qu'une autre molécule, notamment une autre molécule à activité biologique. Lorsque l'ancrage se fait de manière indirecte, la macromolécule peut être greffée chimiquement sur ledit lien, ou interagir de manière physico-chimique avec ledit lien, en particulier lorsque ledit lien intermédiaire est une molécule à activité biologique reconnaissant et interagissant avec ladite macromolécule.

Dans un mode de réalisation, la macromolécule et ledit lien sont tous deux des molécules à activité biologique qui interagissent telles qu'antigène et anticorps respectivement, acides nucléiques complémentaires ou lipides. Dans ces cas, la fixation non covalente de la macromolécule consiste en une liaison de type antigène-anticorps, ligand-récepteur, hybridation entre fragments d'acides nucléiques complémentaires ou interaction hydrophobe ou hydrophile entre lipides.

On met ainsi à profit la très grande spécificité et la très grande sélectivité de certaines réactions biologiques, notamment les réactions antigènes/anticorps, les réactions d'hybridation d'ADN ou d'ARN, les réactions interprotéines ou de type avidine/streptavidine/biotine, de même que les réactions des ligands et de leurs récepteurs.

Ainsi, pour réaliser l'ancrage direct ou indirect de la macromolécule sur la surface S on peut utiliser une surface solide présentant certaines spécificités. Il est en particulier possible d'utiliser certaines surfaces prétraitées permettant de fixer certaines protéines ou de l'ADN, qu'il ait été ou non modifié.

De telles surfaces sont commercialement disponibles (Covalink, Costar, Estapor, Bangs, Dynal par exemple) sous différentes formes présentant à leur suface des groupements COOH, NH₂ ou OH par exemple.

On peut alors fonctionnaliser l'ADN avec un groupement réactif, amine par exemple, et procéder à une réaction avec ces surfaces. Ces méthodes nécessitent cependant une fonctionnalisation particulière de l'ADN à fixer.

On a également décrit une technique permettant l'ancrage sans traitement préalable de l'ADN. Ce procédé consiste à faire réagir un phosphate libre de l'extrémité 5' de la molécule d'ADN avec une amine secondaire de la surface (surface NH Covalink).

L'ancrage par adsorption peut se faire par adsorption de l'extrémité de la molécule en contrôlant la charge surfacique à l'aide du pH, de la teneur ionique du milieu ou de l'application d'une tension électrique sur la surface compte-tenu des différences d'adsorption entre les extrémités de la molécule et sa partie intermédiaire. On a ainsi ancré à titre d'exemple des molécules d'ADN non fonctionnalisées sur des surfaces recouvertes de molécules terminées par un groupe vinyl ou amine telles que des molécules de polylysine ou des surfaces diverses telles que du verre, recouvertes de molécules du type silane terminées par des groupements vinyl ou amine ou encore des lamelles de verre préalablement nettoyées dans un bain d'acide. Dans ce dernier cas, la surface du verre présente en fait des groupes SiOH.

On peut aussi fonctionnaliser l'ADN avec un premier groupement réactif ou une protéine P₀ pour la faire réagir avec une surface recouverte d'un deuxième groupement réactif ou d'une protéine P₁, susceptibles de réagir spécifiquement entre eux (ou elles) respectivement, c'est-à-dire par exemple, P₁ avec P₀. Le couple P₀/P₁ peut être un couple de type biotine/streptavidine (Zimmermann et Cox) ou digoxigénine/anticorps dirigé contre la digoxigénine (anti-DIG), par exemple (Smith et al., Science 258, 1122 (1992)).

De préférence, les surfaces d'ancrage présentent un bas taux de fluorescence pour ne pas gêner la détection des molécules après leur alignement, en particulier si celle-ci se fait par fluorescence.

On utilise de préférence un support solide présentant dans les conditions de réaction une surface ayant une affinité pour une partie de la macromolécule seulement, le reste de celle-ci restant librement en solution.

Dans un mode de réalisation, on utilise un support solide présentant en surface au moins une couche d'un composé organique présentant, à l'extérieur de la couche, un groupement exposé ayant une affinité pour un type de molécule à activité biologique qui peut être ladite macromolécule elle-même ou une molécule reconnaissant et/ou interagissant avec elle.

Le support peut donc présenter une surface recouverte d'un groupement réactif ou d'une molécule à activité biologique.

Par "affinité", il faut entendre aussi bien une réactivité chimique qu'une adsorption d'un type quelconque, ceci dans les conditions éventuelles de fixation des molécules sur le groupement exposé modifié ou non.

Dans un mode de réalisation, la surface est essentiellement compacte, c'est-à-dire qu'elle limite l'accès de la macromolécule à activité biologique aux couches inférieures et/ou au support, ceci afin de minimiser les interactions non-spécifiques, et les autres éléments de la couche ne présentent que peu ou pas d'affinité pour lesdites macromolécules.

On peut aussi utiliser des surfaces recouvertes d'un groupement exposé réactif (par exemple NH₂, COOH, OH, CHO) ou d'une macromolécule à activité biologique (par exemple : des proteines, telles la streptavidine ou des anticorps, des acides nucléiques tels des oligonucléotides, des lipides des polysaccharides et leurs dérivés) capable de fixer une partie éventuellement modifiée de la molécule.

Ainsi des surfaces recouvertes de streptavidine ou d'un anticorps suivant des procédés connus ("Chemistry of Protein Conjugation and Cross-linking", S.C. Wong, CRC Press (1991)) sont capables de fixer une macromolécule présentant en un site particulier une biotine ou un antigène.

De même des surfaces traitées de manière à présenter des oligonucléotides simple-brin peuvent servir pour y ancrer des ADN/ARN possédant une séquence complémentaire.

Parmi les surfaces comportant un groupement réactif exposé, on cite celles sur lesquelles le groupement exposé est un groupement -COOH, -CHO, NH₂, -OH, ou un groupement vinyl comportant une double liaison -CH=CH₂ utilisée telle quelle ou qui peut être activée pour donner notamment les groupes -CHO, -COOH, -NH₂ ou OH.

Les supports à surfaces hautement spécifiques peuvent être obtenus par la mise en oeuvre de divers procédés. On peut citer à titre d'exemple :
(A) une couche de polymère carboné, éventuellement branché, d'au moins 1 nm d'épaisseur présentant des groupements réactifs tels que définis ci-après et
(B) des surfaces obtenues par dépôt ou ancrage sur un support solide d'une ou plusieurs couches moléculaires, celles-ci peuvent être obtenues par la formation de couches successives fixées par liaisons non-covalentes, à type d'exemple non limitatif, les films de Langmuir-Blodgett, ou par auto-assemblage moléculaire, ceci permettant la formation d'une couche fixée par liaison covalente.

Dans le premier cas, la surface peut être obtenue par polymérisation d'au moins un monomère générant en surface du polymère ledit groupement exposé, ou bien par dépolymérisation partielle de la surface d'un polymère pour générer ledit groupement exposé, ou encore par dépôt de polymère.

Dans ce procédé, le polymère formé présente des liaisons vyniles tel un dérivé polyénique, notamment des surfaces de type caoutchouc synthétique, tel que le polybutadiène, le polyisoprène ou le caoutchouc naturel.

Dans le deuxième cas, la surface hautement spécifique comporte :
- sur un support, une couche sensiblement monomoléculaire d'un composé organique de structure allongée ayant au moins :
   . un groupement de fixation présentant une affinité pour le support, et
   . un groupement exposé n'ayant pas ou peu d'affinité pour ledit support et ledit groupement de fixation dans les conditions de fixation, mais présentant éventuellement, après une modification chimique suivant la fixation, une affinité pour un type de molécule biologique.

La fixation peut être tout d'abord de type non-covalent, notamment de type hydrophile/hydrophile et hydrophobe/hydrophobe, comme dans les films de Langmuir-Blodgett (K.B. Blodgett, J. Am. Chem. Soc. 57, 1007 (1935).

Dans ce cas, le groupement exposé ou le groupement de fixation seront, soit hydrophiles, soit hydrophobes, notamment des groupements alkyles ou halogénoalkyles tels que CH₃, CF₃, CHF₃, CH₂F, l'autre groupement étant hydrophile.

La fixation peut être également de type covalent, le groupement de fixation va alors réagir chimiquement sur le support.

Certaines surfaces de structure approchante ont déjà été mentionnées dans le domaine électronique, notamment lorsque les fixations sont covalentes, L. Netzer et J. Sagiv, J. Am. Chem. Soc. 105, 674 (1983) et US-A-4 539 061.

Parmi les groupements de fixation, il faut citer plus particulièrement les groupements de type alkoxyde de métal ou de semiconducteur, par exemple silane, notamment chlorosilane, silanol, méthoxy et éthoxysilane, silazane, ainsi que les groupements phosphate, hydroxy, hydrazide, hydrazine, amine, amide, diazonium, pyridine, sulfate, sulfonique, carboxylique, boronique, halogène, halogénure d'acide, aldéhyde.

Tout particulièrement, comme groupement de fixation on préfère utiliser des groupements susceptibles de réagir transversalement avec un groupe équivalent, voisin, pour fournir les liaisons transversales, par exemple il s'agira de dérivés de type alkoxyde de métal ou de semiconducteur, par exemple silane, notamment dichlorosilane, trichlorosilane, diméthoxysilane ou diéthoxysilane et triméthoxy ou triéthoxysilane.

Le choix du groupement de fixation dépend évidemment de la nature du support, les groupements de type silane sont bien adaptés pour la fixation covalente sur le verre et la silice.

Pour ce qui concerne les groupements exposés, et quelle que soit la surface, ils sont choisis de préférence parmi les groupements éthyléniques, acétyléniques ou des radicaux aromatiques, les amines primaires, tertiaires ou secondaires, les esters, les nitriles, les aldéhydes, les halogènes. Mais il pourra s'agir tout particulièrement du groupement vinyl ; en effet, celui-ci peut être soit modifié chimiquement après fixation pour conduire, par exemple, à un groupement carboxylique ou des dérivés de groupements carboxyliques tels que des groupements alcools, aldéhydes, cétones, acides, amines primaires, secondaires ou tertiaires, soit conduire à un ancrage direct pH dépendant des macromolécules biologiques telles que acides nucléiques et protéines sans modification chimique de la surface ou des macromolécules.

De préférence, les chaînes reliant le groupement exposé au groupement de fixation sont des chaines comportant au moins 1 atome de carbone, de préférence plus de 6 et en général de 3 à 30 atomes de carbone.

Pour ce qui concerne le support lui-même, on préfère utiliser de façon générale, du verre, du silicium oxydé en surface, un polymère ou de l'or avec ou sans prétraitement de la surface.

On peut avantageusement utiliser, dans le cas du verre ou de la silice, les techniques connues de fonctionnalisation de surface utilisant des dérivés silanes, par exemple : Si-OH + Cl₃-Si-R-CH=CH₂ donne Si-O-Si-R-CH=CH₂, R consistant par exemple en (CH₂)₄. Une telle réaction est connue dans la littérature, avec utilisation de solvants ultra-purs. La réaction conduit à un tapis de molécules présentant leur extrémité C=C à la surface exposée à l'extérieur.

Dans le cas de l'or, celui-ci étant éventuellement sous la forme d'une couche mince sur un substrat, les techniques connues de fonctionnalisation de surface utilisent des dérivés thiols, par exemple : Au + HS-R-CH=CH₂ donne Au-S-R-CH=CH₂, R consistant par exemple en (CH₂)₄. Une telle réaction est décrite en milieu liquide et conduit, de même que la réaction précédente trichlorosilane-silice, à un tapis de molécules présentant leur extrémité C=C à la surface exposée à l'extérieur.

Les surfaces ainsi obtenues sont, de préférence, revêtues d'une macromolécule à activité biologique choisie parmi :
- les protéines,
- les acides nucléiques,
- les lipides,
- les polysaccharides et leurs dérivés.

Parmi les protéines, il faut citer les antigènes et les anticorps, les ligands, les récepteurs, mais également des produits de type avidine ou streptavidine ainsi que les dérivés de ces composés.

Parmi les ARN et les ADN, il faut également citer les dérivés α, β ainsi que les dérivés thio et les composés mixtes tels que les PNA.

On peut également fixer des composés mixtes tels que les glycopeptides et les lipopolysaccharides par exemple, ou bien d'autres éléments tels que virus, cellules notamment, ou composés chimiques tels que la biotine.

La fixation des macromolécules biologiques peut être covalente ou non-covalente, par exemple par adsorption, liaisons hydrogènes, interactions hydrophobes, ioniques, par exemple, auquel cas on pourra procéder avantageusement à un pontage ("cross-linking") entre les molécules greffées par les méthodes connues ("Chemistry of Protein Conjugation and Cross-linking", S.C. Wong, CRC Press (1991)) et ceci afin de renforcer leur cohésion.

Comme cela a été mentionné précédemment, il est possible d'avoir un groupement exposé qui permet la réaction directe avec les molécules à activité biologique, mais il est également possible de prévoir que le groupement exposé est traité, après fixation, pour être transformé, comme cela a été indiqué précédemment, en un radical hydroxy, amine, alcool, aldéhyde, cétone, COOH ou dérivé de ces groupements avant la fixation de la molécule biologique.

Lorsque de tels groupements ont été exposés, les techniques de fixation des protéines et/ou de l'ADN, par exemple, sont connues, il s'agit en effet de réactions mises en oeuvre pour des surfaces qui sont déjà utilisées dans le cadre des analyses biologiques, notamment pour les surfaces Costar et les surfaces Nunc ou des microbilles telles qu'Estapor, Bang et Dynal par exemple, sur lesquelles on ancre des molécules d'intérêt biologique, ADN, ARN, PNA, protéines ou anticorps par exemple.

Dans le cas où le groupement exposé est un radical -CH=CH₂ qui est nommé ci-après "surface C=C" ou "surface à liaison éthylénique", il n'existe pas de document mentionnant l'ancrage direct, en particulier de l'ADN ou des protéines. Il a été démontré que ces surfaces présentent une réactivité fortement pH dépendante. Cette particularité permet d'ancrer les acides nucléiques ou les protéines en utilisant des zones de pH et souvent avec une vitesse de réaction qui peut être contrôlée par le pH.

On peut réaliser l'ancrage de l'ADN par son extrémité sur une surface présentant des groupements à double liaison éthylénique en mettant l'ADN en présence de la surface à un pH inférieur à 8.

En particulier la réaction est conduite à un pH compris entre 5 et 6 puis est stoppée à pH 8.

Ainsi, pour l'ADN à pH 5,5, la réaction d'ancrage est totale en une heure (si pas limitée par la diffusion) et se produit par les extrémités. A pH 8, par contre, l'accrochage est très faible (vitesse de réaction de 5 à 6 ordres de grandeur plus faibles). Cet effet d'accrochage pH dépendant et spécifique des extrémités, présente une amélioration par rapport aux autres surfaces qui nécessitent une fonctionnalisation de l'ADN (biotine, DIG, NHS, ...) ou des réactifs spécifiques (carbodiimide, diméthyle pimélidate) qui réalisent une liaison peptidique ou phosphorimide entre - NH₂ et -COOH ou -POOH.

On peut aussi réaliser l'ancrage d'ADN par adsorption de ses extrémités seulement sur une surface recouverte de polylysine ou d'un groupement silane terminé par un groupe amine.

Pour réaliser l'ancrage de l'ADN par son extrémité sur une surface recouverte par un groupement amine on met l'ADN en présence de la surface à pH entre 8 et 10.

On peut réaliser l'ancrage d'ADN par son extrémité sur une surface de verre prétraitée auparavant dans un bain d'acide, en mettant l'ADN en présence de ladite surface à pH entre 5 et 8.

De même ces surfaces peuvent ancrer des protéines directement (protéine A, anti-DIG, anticorps, streptavidine, etc.). Il a été observé que (i) l'activité de la molécule peut être préservée et (ii) que la réactivité de la surface préparée (initialement C=C) est totalement occultée pour faire place à la seule réactivité de la molécule d'intérêt. 11 est donc possible, à partir d'une réactivité initiale relativement large, de passer à une surface possédant une réactivité très hautement spécifique, par exemple celle de sites spécifiques sur une protéine.

En ancrant un anticorps spécifique sur la surface (par exemple anti-DIG), on crée une surface dont la réactivité est limitée à l'antigène (par exemple le groupement DIG). Ceci indique que les groupements chimiques initiaux ont tous été occultés par les anticorps greffés.

On peut aussi greffer sur les surfaces réactives (chimiquement ou biochimiquement) d'autres molécules à activité biologique, notamment des virus ou d'autres composants : membranes, récepteurs membranaires, polysaccharides, PNA, notamment.

Il est également possible de fixer le produit d'une réaction d'intérêt biologique (par exemple la PCR) sur les surfaces préparées.

L'appareillage selon la présente invention permet la mise en évidence et/ou la quantification de molécules biologiques, mais également la mesure de distance intramoléculaire, la séparation de certaines molécules biologiques, notamment un prélèvement par mise en oeuvre des techniques de couplage antigène/anticorps et/ou ADN/ARN.

En particulier, l'appareillage selon la présente invention peut être utilisé dans un procédé de mise en évidence d'une macromolécule consistant en une séquence d'ADN ou d'une protéine dans un échantillon, dans lequel :
- on met l'échantillon correspondant au solvant A, dans lequel ladite macromolécule est en solution, en contact avec la surface du support dans des conditions de formation d'un hybride ADN/ADN, ADN/ARN ou de formation du produit de réaction protéine/protéine,
- l'hybride ou une macromolécule de marquage de l'hybride ou du produit de réaction étant ancré en une partie, le reste étant libre en solution, on l'étire par déplacement du ménisque créé par le contact du solvant avec la surface pour orienter les hybrides ou lesdites macromolécules de marquage et on effectue la mesure ou l'observation des hybrides ou desdites macromolécules de marquage ainsi orientés.

Le passage du ménisque, en étirant linéairement les molécules, sous forme de bâtonnets, les rend plus facilement détectables si elles sont marquées. Par ailleurs, ces molécules allongées sont stables à l'air libre et peuvent être observées même après plusieurs mois, sans présenter de dégradation apparente.

Lors d'une réhydratation, les molécules d'ADN peuvent rester adsorbées et allongées. De plus, il est possible de procéder à une hybridation sur la molécule allongée.

De plus, présentant un signal corrélé et d'orientation uniforme de par leur étirement, ces molécules sont distinctes du bruit environnant. Il est donc facile d'ignorer les poussières, les inhomogénéités, qui ne présentent pas de corrélation spatiale particulière. L'alignement est aussi intéressant car en solution, les molécules en pelote fluctuent thermiquement, ce qui entraine des variations très importantes de leur signal de fluorescence recueilli de préférence avec une faible profondeur de champs et limite leur observation. L'appareillage selon la présente invention permet donc l'observation de molécules isolées avec un très grand rapport signal sur bruit. (S/N).

Il est remarquable que ce rapport soit indépendant du nombre de molécules ancrées. Le rapport S/N posé par la détection d'une molécule est le même que pour 10 000. De plus cette technique d'étirement permet de discriminer aisément entre des molécules de longueurs variées.

Les molécules étirées peuvent être révélées par différentes méthodes enzymologiques ou autres, telles la fluorescence ou l'utilisation de sondes chaudes ou froides. Leur détection peut se faire par mesure d'un signal global (par exemple la fluorescence) ou par l'observation individuelle des molécules par microscopie optique à fluorescence, microscopie électronique, méthodes à sondes locales (STM, AFM, etc.).

Ainsi de façon générale, pour la mise en évidence, la séparation et/ou le dosage d'une molécule dans un échantillon, on utilise une surface capable de fixer spécifiquement ladite molécule, et la mise en évidence, la séparation ou le dosage sont effectués grâce à un réactif fluorescent ou non détectant la présence de la molécule fixée.

Parmi les réactifs on distingue les réactifs fluorescents et les réactifs non-fluorescents.

Les réactifs fluorescents contiennent des molécules fluorescentes, choisies avec avantage pour être des molécules longues de taille supérieure à 0,1 µm et réagissant de manière spécifique directement ou indirectement avec les surfaces prétraitées. Par exemple, une molécule d'ADN double brin marquée à l'aide de sondes fluorescentes (ethidium bromide, YOYO, nucléotides fluorescents, etc.) pouvant s'ancrer directement par une ou plusieurs extrémités sur une surface présentant éventuellement un groupement de type vinyl, amine ou autre. notamment par un choix judicieux du pH ou de la teneur ionique du milieu ou par application d'une tension électrique sur la surface.

Il est également possible d'utiliser une fonctionnalisation particulière de la molécule (DIG, biotine etc...) pour l'ancrer en un ou plusieurs points sur une surface présentant des sites complémentaires (anti-DIG, streptavidine, etc...).

Les réactifs non-fluorescents permettant la détection de molécules préalablement alignées selon la présente invention, peuvent consister notamment en des billes ou microparticules ancrées par l'intermédiaire d'une autre molécule fixée de manière spécifique directement ou indirectement à la molécule alignée et ne présentant qu'une faible interaction non-spécifique avec la surface.

Selon que la molécule recherchée est détectée directement par fluorescence ou indirectement à l'aide des réactifs ci-dessus, on parlera de "détection directe" ou "par drapeau".

La détection du nombre de molécules alignées peut être réalisée sur un petit nombre de molécules (typiquement 1 à 1 000), par un test physique macroscopique faible bruit ne nécessitant ni microscope électronique ni radioactivité ni nécessairement la PCR.

L'appareillage selon la présente invention est susceptible d'être utilisé par des personnes n'ayant qu'une expérience de laboratoire réduite.

Les techniques d'alignement et de détection peuvent être utilisés pour de nombreuses applications parmi lesquelles, mais sans s'y restreindre :
- l'identification d'un ou plusieurs éléments de séquence d'ADN ou d'ARN que l'on peut utiliser avec avantage pour le diagnostic de pathogènes ou la cartographie physique d'un génome. En particulier, les techniques décrites ci-dessus permettent l'obtention d'une cartographie physique directe sur ADN génomique, sans passer par une étape de clonage. Il est entendu que la molécule peignée étant étirée par rapport à sa longeur crystallographique, on procède à des mesure relatives. Il est ainsi possible de mesurer la taille de fragments d'ADN et la distance entre fragments avec une résolution de l'ordre de 200 nm pour des méthodes optiques ou de l'ordre de 1 nm par l'utilisation de méthodes de champs proche telles que AFM ou STM pour visualiser et mesurer la distance entre sondes sur de l'ADN aligné.
   Ceci conduit naturellement à :
   1) la détection de délétions, additions ou translocations de séquences génomiques, en particulier dans le diagnostic de maladies génétiques (par exemple la myopathie de Duchesne) ;
   2) l'identification de promoteurs de différents gènes par la mesure de la distance entre les séquences régulatrices et celle exprimées ;
   3) la localisation de protéines régulatrices par l'identification de leur position le long de l'ADN ou de la position de leur séquence cible ;
   4) le séquençage partiel ou total par mesure de la distance à l'aide de microscopies à champs proche (par exemple AFM ou STM) entre des sondes hybridées appartenant à une base d'oligonucléotides de longueur donnée.
- la PCR in situ sur des ADN alignés,
- l'amélioration de la sensibilité des techniques d'ELISA avec la possibilité de détecter un faible nombre (éventuellement inférieur à 1 000) de réactions immunologiques.
   5) l'identification de régions de transcription de gènes comme introns et exons, la distance entres les introns et exons et la grandeur des introns et des exons.

Ainsi, on peut procéder à une cartographie physique directement sur un ADN génomique sans passer par une étape de clonage. L'ADN génomique est extrait, purifié, éventuellement coupé par un ou plusieurs enzymes de restriction puis peigné sur des surfaces selon le procédé de la présente invention.

La position et la taille du gène recherché sur l'ADN génomique sont alors déterminées par hybridation avec des sondes spécifiques dudit gène, notamment extraites de parties de l'ADN complémentaire (cDNA) du produit dudit gène recherché.

De façon similaire en hybridant un ADN génomique peigné, puis dénaturé avec du cDNA total marqué, on identifie la position, la taille et le nombre des exons du gène en question.

La position du gène étant déterminée comme décrit ci-dessus, ou connue, il est alors possible d'identifier par hybridation les séquences flanquantes du gène. Pour cela, on procède avec avantage, par hybridation avec des sondes marquées, provenant par exemple d'une librairie d'oligonucléotides, pour identifier deux ou plusieurs sondes qui s'hybrident de part et d'autre du gène.

A partir de cette détermination, il est alors possible par les techniques de PCR in situ d'amplifier le fragment délimité par les sondes flanquantes qui peuvent servir d'amorces à la réaction, fragment qui peut contenir le gène recherché avec ses régions régulatrices qui peuvent être tissu ou développement spécifique.

L'amplification du gène recherché procède alors par les techniques connues de PCR sur le fragment amplifié comme décrit ci-dessus en utilisant des amorces accessibles par les exons constituant le cDNA.

Par le peignage d'ADN génomique ou autre, il est aussi possible de déterminer par hybridation la présence ou l'absence de séquences régulatrices d'un gène particulier proximal, à partir desquelles on déterminera les familles possibles de protéines régulatrices de ce gène (par exemple : helix-loop-helix, zinc-finger, leucine-zipper).

Les réactions spécifiques entre séquences particulières d'ADN/ARN/PNA et une autre molécule (ADN, ARN, protéine) peuvent se faire avant ou après alignement des molécules selon la présente invention.

Ainsi, dans le cadre du diagnostic génétique et de la cartographie physique, on utilise avec avantage les méthodes connues de FISH (Pinkel et al., Proc. Nat. Acad. Sci. USA 83, 2934 (1986)) pour hybrider des oligonucléotides simple-brin marqués à de l'ADN d'abord aligné, puis dénaturé. La révélation des hybrides se fera par les techniques connues (fluorescences, microbilles etc...) avec une résolution dans la mesure des distances allant de 0,2 µm (en optique) à 1nm (en microscopie à champ proche ; AFM, STM etc...).

Alternativement, on peut d'abord hybrider des ADN marqueurs fluorescents à de l'ADN simple-brin en solution, puis aligner cette construction par l'action du ménisque après l'avoir transformé en ADN double-brin et ancré à une surface adéquate.

On peut aussi utiliser l'appareillage de la présente invention pour la détection de la présence d'un pathogène. A titre d'exemple, on pourra procéder de deux manières différentes selon que la réaction de reconnaissance (hybridation, attachement de protéine) ait lieu avant ou après alignement par le ménisque.

Ainsi, à titre d'exemple, un ou plusieurs oligonucléotides sondes sont ancrées à une ou plusieurs régions d'une surface. L'hybridation de l'ADN potentiellement pathogénique est effectuée in situ dans des conditions stringentes de façon à n'ancrer que les molécules hybridées. Leur détection et quantification s'effectuent après alignement par le ménisque selon la présente invention.

Alternativement, l'ADN potentiellement pathogénique est d'abord aligné, puis dénaturé et hybridé avec un oligonucléotide sonde dans des conditions stringentes. La détection de l'hybride s'effectue alors par les méthodes connues notamment de FISH, comme décrit ci-dessus.

D'une manière similaire, on peut détecter la présence (ou l'absence) d'un petit nombre de molécules, telles des protéines, des lipides, des sucres ou des antigènes. On procèdera avec avantage, à une modification mineure des techniques d'ELISA, la méthode de détection habituelle étant remplacée par la détection d'une molécule fluorescente alignée selon la présente invention et couplée à l'un des réactifs de la réaction d'ELISA.

Par ailleurs, comme mentionné par K.R. Allan et al. (US 84 114), la cartographie génétique peut procéder par une mesure de la taille de fragments d'ADN. Or les techniques originales d'étirement des molécules décrites plus haut (l'étirement par le ménisque) permettent une mesure de la longueur des molécules étirées et cela sur un très petit échantillon (quelques milliers de molécules).

On peut, par exemple, mais sans s'y restreindre, procéder de la manière suivante :
Un échantillon d'ADN est fragmenté (à l'aide d'enzymes de restriction), teinté avec un fluorophore puis ancré sur une surface. Les molécules sont ensuite étirées par le ménisque et la taille des fragments étirés déterminée par microscopie optique à fluorescence avec une résolution et une taille limite de l'ordre de 1 000 bp (0,3 µm).

Dans ce but, mais aussi si l'on veut aligner des molécules très longues (≥ 10µm) on utilisera avec avantage des techniques connues pour limiter la dégradation de longues macromolécules lors de leur manipulation (par cisaillement hydrodynamique).

L'appareillage selon la présente invention peut être utilisé dans un procédé d'alignement et de mise en évidence de l'ADN dans lequel l'ADN est étiré par un procédé d'alignement selon l'invention, puis dénaturé puis hybridé avec des sondes spécifiques pour déterminer la position ou la taille d'une ou plusieurs séquences spécifiques.

L'appareillage selon la présente invention peut également être utilisé pour la mise en oeuvre d'un procédé de cartographie physique d'un gène sur un ADN génomique dans lequel l'ADN est aligné ou mis en évidence selon un procédé de l'invention.

En particulier, la position et la taille du gène recherché sur l'ADN génomique, sont déterminées par hybridation avec des sondes spécifiques dudit gène à cartographier.

L'appareillage de la présente invention permet la mise en oeuvre d'un procédé de diagnostic d'une pathologie liée à la présence ou l'absence d'une séquence d'ADN spécifique de la pathologie dans lequel on utilise un procédé d'alignement selon l'invention.

Enfin, l'appareillage selon la présente invention est utilisé dans un procédé de préparation d'un gène dans lequel on identifie la position dudit gène sur de l'ADN génomique aligné par le procédé selon l'invention à l'aide d'une sonde spécifique dudit gène on amplifie par PCR in situ la séquence dudit gène.

L'appareillage de la présente invention permet donc de mettre en oeuvre un procédé de remplacement d'un gène dans le génome d'une cellule eucaryote par l'insertion ciblée d'un gène étranger préparé selon le procédé de préparation de gène ci-dessus.

L'insertion ciblée peut être effectuée selon les techniques décrites dans WO90/11354 en transfectant des cellules eucaryotes avec un vecteur contenant ledit ADN étranger à insérer flanqué de deux séquences génomiques qui jouxtent le site d'insertion souhaité dans le gène receveur. L'ADN d'insertion peut comporter soit une séquence codante, soit une séquence régulatrice. Les séquences flanquantes sont choisies afin de permettre par recombinaison homologue, selon le cas, soit l'expression de la séquence codante de l'ADN d'insertion sous le contrôle des séquences régulatrices du gène receveur, soit l'expression d'une séquence codante du gène receveur sous le contrôle de séquence régulatrice de l'ADN d'insertion.

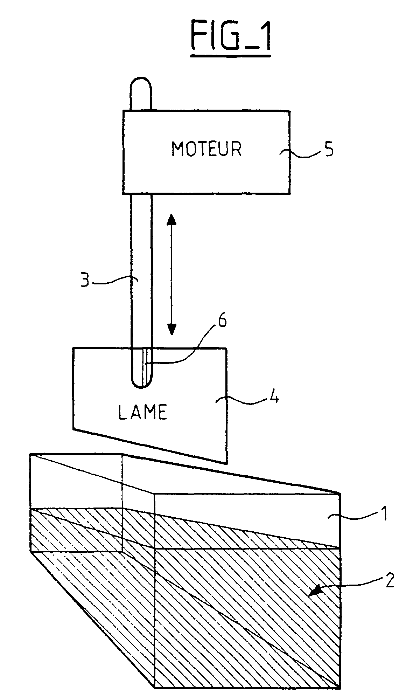

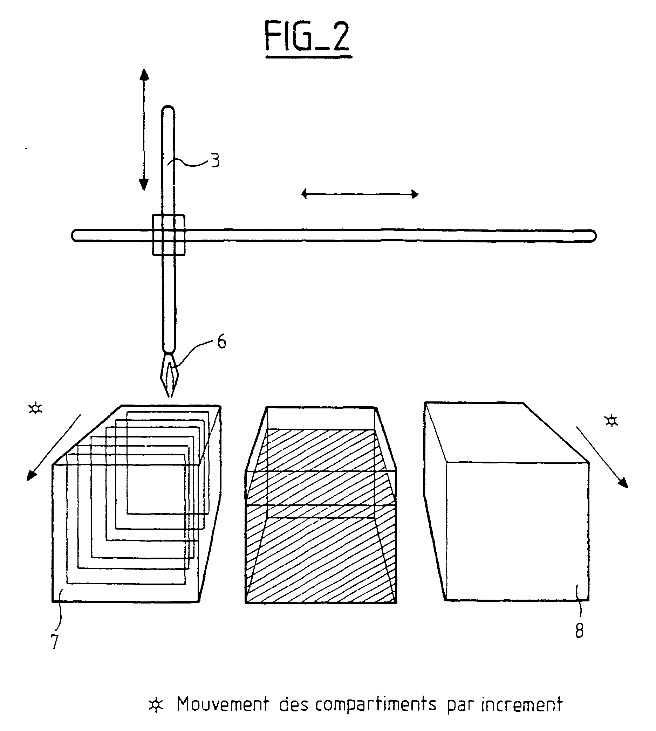

D'autres caractéristiques et avantages de la présente invention apparaitront à la lumière de la description qui va suivre faite en se référant aux figures 1 et 2 annexées sur lesquelles est représenté un appareillage selon l'invention consistant schématiquement en deux parties (voir figure 1) :
- un récipient (1) contenant la solution de molécules à peigner (2),
- une tige verticale (3) comportant des moyens de fixation (6) pouvant supporter une (ou plusieurs) surface(s) (4) sur lesquelles doit avoir lieu le peignage, la tige étant mobile le long d'un axe vertical à l'aplomb du récipient (1),
- un moteur (5) qui permet de déplacer la tige en translation verticale à l'aplomb du récipient (1).

Le ménisque formé par l'interface surface S/solution/air est rectiligne. Il reste fixe lorsque la tige verticale est mise en mouvement et que la surface est progressivement retirée de la solution, ou que la solution est progressivement retirée du réservoir. La direction d'étirement, perpendiculaire au ménisque, est donc parallèle à l'axe vertical, et uniforme sur toute la surface.

L'ensemble de ces deux parties constitue la base d'un appareillage que l'on désigne sous le nom d' "*ascenseur*", en référence au mouvement de translation vertical des lames.

Tel que représenté figure 2, l'appareillage comprend avantageusement un premier compartiment de lames à incuber (7), et un deuxième compartiment receptionnant les lames incubées sur lesquelles l'ADN est peigné (8). La tige (3) est équipée à son extrémité basse d'un dispositif de fixation simple des lames (6) (type "mâchoire" ou pince), et il suffit de lui ajouter un degré de liberté de translation horizontale pour lui permettre de se présenter successivement à l'aplomb des compartiments (7) et (8) et du récipient (1).

Un cycle de "peignage" consiste en :
(i) saisie des lames à incuber,
(ii) translation au-dessus du réservoir,
(iii) descente dans le réservoir et incubation,
(iv) sortie des lames du réservoir,
(v) translation au-dessus du compartiment de stockage,
(vi) dépôt des lames dans un deuxième compartiment,
(vii) retour à l'étape (i).

Il est possible d'utiliser comme compartiment de stockage des lames à incuber, le même récipient qui sert à entreposer les lames lors de leur traitement physico-chimique, simplifiant de la sorte les manipulations confiées à l'utilisateur.

Les compartiments (7) et (8) sont équipés de moyens permettant leur mouvement en translation horizontale de sorte que lorsque la tige (3) arrive à l'aplomb de ces compartiments, le compartiment (7) présente la lame à incuber, et le compartiment (8) présente un espace vide pour recevoir la lame sur laquelle les macromolécules sont alignées.

### EXEMPLE 1

### Matériels et méthodes

L'ADN-λ et l'anticorps monoclonal (Anti-DIG) proviennent de Boehringer-Mannheim. Les trichlorosilanes proviennent de Roth-Sochiel. Les sondes nucléiques fluorescentes (YOYO1, YOYO3 et POPO1) proviennent de Molecular Probes. Les lamelles de verre ultrapropres proviennent de Erie Scientific (Lamelles (ESCO). Les sondes possèdent un groupement réactif (DIG, biotine, etc.) capable de s'ancrer de manière spécifique à une surface selon la présente invention (ayant par exemple comme site d'ancrage un anticorps anti-DIG ou la streptavidine). La détection de la réaction d'ancrage se fait directement par détection de la fluorescence de la molécule d'ADN teintée par des molécules fluorescentes (ethidium bromide, YOYO, nucléotides fluorescents).

### Traitement de surface

Des lamelles de verre sont nettoyées pendant une heure par irradiation UV sous atmosphère d'oxygène (par formation d'ozone). Elles sont ensuite immédiatement déposées dans un dessicateur préalablement purgé de traces d'eau par un courant d'argon. Un volume d'environ 100 à 500 µl du trichlorosilane approprié (H₂C=CH-(CH₂)_{N}-SiCl₃ est introduit dans le dessicateur, d'où les surfaces sont retirées après environ 12 heures (*n* = 6) ou 1 heure (*n* = 1). Au sortir les surfaces sont nettes et non-mouillantes.

Les groupes fonctionnels de ces surfaces double liaison (H₂C=CH-) peuvent être transformés en groupements carboxyles (-COOH) en trempant les lamelles traitées, comme décrit précédemment, pendant une dizaine de minutes dans une solution de 25 mg KM*n*O₄, 750 mg NaIO₄ dans 1 l d'eau, puis en les rinçant trois fois dans de l'eau ultrapure.

Les lamelles ainsi fonctionnalisées peuvent réagir avec des protéines. Un volume de 300 µl d'une solution aqueuse (20 µg/ml) de protéines (protéine A, streptavidine, etc.) est déposé sur une lamelle fonctionnalisée en groupement (H₂C=CH-). Cette lamelle est incubée environ deux heures à température ambiante, puis rincée trois fois dans de l'eau ultrapure. Les surfaces ainsi traitées sont nettes et mouillantes. Les surfaces traitées à la protéine A peuvent ensuite réagir avec un anticorps, par exemple anti-DIG, par incubation dans une solution de 20 µg/ml d'anticorps.

Par ailleurs, sur les surfaces présentant des groupements carboxyles, on peut greffer des oligonucléotides présentant une extrémité amine (-NH₂), 200µl d'une solution de MES (50 mM, pH 55), Carbodiimide (1mg/ml) et 5µl d'oligo-aminé (10 pmole/140 µl) sont déposés sur une surface carboxylée et incubés environ 8 heures à température ambiante. La lamelle est finallement rincée trois fois dans NaOH (0.4 M) puis quatre fois dans de l'eau ultrapure. Les lamelles ainsi préparées peuvent hybrider des ADN complémentaires de l'oligonucléotide ancré.

### Ancrage d'ADN natif sur surface double liaison

Une lamelle prétraitée (H₂C=CH-) est immergée dans une solution de 5 à 10 ml d'ADN-λ marqué par fluorescence (YOYO1, POPO1 ou YOYO3, mais sans terminaison particulière) de concentration variable et dans différents tampons (nombre total de molécules < 10⁷). La préparation est incubée environ 10 à 15 minutes à température ambiante dans une atmosphère saturée en vapeur d'eau. Dans un tampon de 0,05 M MES (pH = 5,5), on observe un ancrage quasi-général des molécules d'ADN. Par contre dans un tampon de 0,01 M Tris (pH = 8) il n'y a presqu'aucune molécule d'ancrée (rapport > 10⁶). Cette dépendance peut permettre le contrôle de l'activation/désactivation des surfaces (vis-à-vis de l'ADN) par l'intermédiaire du pH.

Des conditions identiques sont utilisées pour ancrer un YAC de levures contenant un insérat de plusieurs gènes humains d'une largeur comprise entre 700 Kb et 1 Mégabase.

### Alignement et détection de l'ancrage par l'action du ménisque

L'action du ménisque sur la molécule est limitée au voisinage immédiat de celui-ci. La partie de la molécule en solution devant le ménisque fluctue librement et la partie laissée collée à la surface derrière le ménisque est alignée dans la direction de déplacement du ménisque. Le taux d'extension de la molécule est donc uniforme et indépendant de sa taille.

En transférant la préparation précédente dans une atmosphère sèche, la lame est retirée de la solution et les molécules d'ADN, ancrées à la surface sont étirées perpendiculairement au ménisque. La force capillaire sur la molécule d'ADN (quelques dizaines de picoNewtons) est en effet suffisante pour étirer complètement la molécule (plus grande que les forces d'élasticité entropique), mais trop faible pour briser la liaison entre l'extrémité de la molécule et la surface traitée. L'ADN étant marqué par fluorescence, on observe individuellement et aisément les molécules étirées. L'ancrage entre la surface et l'ADN étant limité aux extrémités, on a pu aussi bien étirer des ADN de phage λ ou de YAC (longueur totale supérieure à 400 µm).

### Ancrage et détection spécifiques

En traitant les surfaces comme décrit précédemment avec un anticorps monoclonal spécifique, on peut contrôler très précisément leur spécificité. Ainsi, on a testé la spécificité de surfaces traitées anti-DIG vis-à-vis d'ADN-λ hybridés avec un oligonucléotide complémentaire d'une des extrémités Cos et possédant un groupement digoxigénine (DIG) et vis-à-vis d'ADN non hybridés. Dans le premier cas, on a observé une extension, par action du ménisque, quasi-générale des molécules ancrées. Dans le second cas, on n'a observé que quelques molécules d'ADN (< 10) ancrées dans tout l'échantillon.

Des ADN-λ ont aussi été hybridés avec des oligonucléotides complémentaires d'une des extrémités COS et fixés sur des surfaces carboxylées, comme décrit ci-dessus. Pour visualiser l'hybridation il faut se mettre dans des conditions de faible stringence (eau pure à 40°C). Dans des conditions de stringence forte (haute salinité) la fluorescence des sondes YOYO1 disparaît et les ADN hybridés ne peuvent être vus. Les ADN ainsi hybridés ont aussi pu être alignés par passage du ménisque.

### Dépendance de l'étirement sur le traitement de surface

L'histogramme des longueurs d'ADN-λ, greffés sur des surfaces différentes, puis alignés par passage du ménisque montre un pic bien défini, mais différent pour les différentes surfaces. Ainsi sur des surfaces recouvertes d'un silane se terminant par un groupement vinyl l'ADN est étiré jusqu'à environ 20 à 24 µm et sur du verre propre à environ 19 µm et sur des surfaces de verre préalablement traitées à la protéine A et à l'anti-DIG l'étirement est de 16 microns..

L'étirement dépend donc du traitement de surface. Sur des surfaces hydrophobiques l'étirement sera beaucoup plus long (vinyl, NH₂). Sur des surfaces hydrophyliques il n'y a quasiment pas d'étirement.

### EXEMPLE 2

### Peignage de molécules d'ADN sur différentes surfaces

On a observé le peignage moléculaire de l'ADN sur des surfaces de verre traitées de différentes façons. On joue sur la différence d'adsorption entre les extrémités de la molécule et le reste de celle-ci. En adsorbant des polymères chargés positivement sur une surface de verre on favorise une adsorption des molécules d'ADN chargées négativement, cependant lorsque cette charge est grande la molécule d'ADN est collée sur toute sa longueur et le peignage est impossible. Mais il est possible de modifier la charge des polymères adsorbés sur le verre en modifiant les conditions de pH (ou de salinité), en effet, les charges positives sont portées par exemple par des groupes *NH*_{*2*} qui passent à l'état protonné *NH+*_{*3*} pour un pH inférieur au pK de la base correspondante. En pH basique les charges disparaissent et la surface n'attire plus l'ADN. En contrôlant finement le pH on a observé que les molécules d'ADN en solution passaient d'un état où elles sont complètement collées à la surface à une phase intermédiaire où elles ne sont ancrées que par leurs extrémités puis à une phase où la surface ne présente plus d'affinité pour l'ADN. Dans la phase intermédiaire le peignage moléculaire est réalisable.

On a étudié des surfaces recouvertes d'un silane terminé par un groupement *NH*_{*2*} pour lesquelles on observe un collage total à pH < 8, un peignage pour 8.5 < *pH <* 9.5. Le nombre de molécules peignées est maximum à pH = 8.5 il est divisé par 2 à pH = 9 et par 4 à pH = 9.5. On a aussi déterminé l'extension relative sur cette surface qui correspond à 1.26 sur l'histogramme.

On a aussi regardé des surfaces recouvertes de polylysine qui présentent des caractéristiques d'accrochages similaires en pH : domaine de peignage 8.5 et présentent une extension relative plus faible : 1.08.

Finalement, on a retrouvé le même comportement sur des surfaces de verre fraîchement nettoyées dans un mélange eau oxygénée / acide sulfurique concentré. Ces surfaces sont très mouillantes et se polluent rapidement, cependant, on a observé un domaine de peignage entre 5.5 < pH < 7.4 tandis que le domaine d'adsorption forte se situe à *pH = 4.5.* L'extension relative des molécules correspond à 1.12.

### Alignement uniforme et directionel de YAC

1 µg de YAC préalablement teint dans son bloc d'agarose à l'aide d'une sonde fluorescente YOYO1 est chauffé à 68°C, agarasé, puis dilué dans 10 ml de MES (50mM pH 5.5). Deux lamelles silanisées (surfaces C=C) sont incubées pendant 15 minutes dans cette solution puis retirées à environ 170 µm/sec. Les molécules de YAC sont toutes alignées de manière quasi parallèle à la direction du retrait des lamelles (Fig. 9). L'intégrité des molécules ainsi alignées est meilleure que par évaporation après déposition entre deux lamelles.

### Hybridation d'un cosmide sur un YAC peigné

Un YAC marqué au YOYO comme décrit précédemment est ancré sur une surface C=C puis aligné par le ménisque, lors du retrait de la lamelle de la solution. Les sondes (cosmides) sont marquées par incorporation d'un nucléotide biotynilé par la technique de "randon priming". Les sondes marquées (100 ng) et 5 µg d'ADN de sperme de saumon soniqué (=500 bps) sont purifiées par précipitation dans Na-acétate et éthanol, puis dénaturées dans une solution de formamide (50 % formamide, 2% FFC, 10 % sulfate de dextran).

Les YAC peignés sont dénaturés avec 120 µl de solution dénaturante (formamide 70 %, 2 x SSC) sur une plaque chauffante à 86°C pendant 6 minutes. Les sondes (20 ng) préalablement dénaturées sont déposées sur la lamelle dans une solution d'hybridation (formamide 55 %, 2 x SSC, 10 % dextran sulfate) recouvertes d'une lamelle qui est scellée avec du caoutchouc liquide (rubber cement). L'hybridation est réalisée une nuit à 37°C en chambre humide.

La révélation des hybrides se fait suivant les protocoles connus pour les hybridations in situ sur chromosomes décondensés (D. Pinkel et al., PNAS USA 83, 2934 (1986) et PNAS USA 85, 9138 (1988)).

En microscopie, en fluorescence on observe alors des segments hybridés tels celui montré dans la Fig. 10. Cet exemple démontre la possibilité de détecter la présence d'un gène sur une molécule d'ADN, qui peut être utilisé à des fins de diagnostic ou de cartographie physique du génome.

## Revendications

1. Appareillage d'alignement parallèle de macromolécules sur la surface S d'un support solide **caractérisé en ce qu'**il comporte
- un récipient (1) destiné à recevoir une solution (2) contenant les macromolécules à aligner,
- un support solide de surface S configurée pour fixer lesdites macromolécules,
- des moyens d'immersion dudit support (4) dans ladite solution (2) pour former un ménisque créé par l'interface triple surface S/solution/air, et
- des moyens de déplacement rectiligne relatif de la surface S et de la surface de ladite solution pour permettre l'établissement d'un alignement parallèle des macromolécules sur la surface.

2. Appareillage selon la revendication 1, **caractérisé en que** lesdits moyens d'immersion du support et lesdits moyens de déplacement relatif du ménisque et de la surface S consistent en des mêmes moyens de translation verticale (3 à 6) de ladite surface S à l'aplomb de la surface de ladite solution.

3. Appareillage selon la revendication 1 ou 2, **caractérisé en ce que** lesdits moyens de translation verticale de la surface S comprennent :
- des moyens de préhension (6) du support solide, et
- des moyens de guidage et moteur (5) pour provoquer la translation desdits moyens de préhension le long d'un axe vertical (3) disposé à l'aplomb du récipient.

4. Appareillage selon la revendication 3, **caractérisé en ce que** lesdits moyens de translation verticale de la surface S comprennent :
- des moyens de préhension du support solide consistant en une tige verticale (3) disposée à l'aplomb dudit récipient et supportant des moyens de pincement (6) du support solide, et
- des moyens de guidage et moteur pour commander la translation verticale de la tige entre une position basse d'immersion et une position haute de retrait.

5. Appareillage selon l'une des revendications 1 à 4, **caractérisé en ce que** l'appareillage comporte en outre :
- un premier compartiment de stockage de supports solides, notamment de lames avant immersion dans ledit récipient, et
- un deuxième compartiment de stockage de supports solides, notamment de lames sur lesquelles les macromolécules sont alignées après leur retrait dudit récipient.

6. Appareillage selon les revendications 4 et 5, **caractérisé en ce qu'**il comporte en outre des moyens pour présenter successivement lesdits supports solides à l'aplomb dudit premier compartiment dudit récipient et dudit deuxième compartiment.

7. Appareillage selon les revendications 4 et 6, **caractérisé en ce que** lorsque lesdits compartiments et ledit récipient sont alignés, l'appareillage comporte en outre des moyens de guidage et moteur pour provoquer la translation horizontale de la tige quand elle est en position haute pour permettre de présenter lesdits supports solides à l'aplomb desdits compartiments et dudit récipient.

8. Appareillage selon l'une des revendications 1 à 7, **caractérisé en ce qu'**il comporte un dispositif de contrôle du pH dans la solution contenue dans ledit récipient.

9. Appareillage selon l'une des revendications 1 à 8, **caractérisé en ce que** lesdits moyens de déplacement du ménisque permettent le déplacement du ménisque à une vitesse de 10 à 100 µm/sec.

10. Appareillage selon l'une des revendications 1 à 9, **caractérisé en ce que** le volume dudit récipient est de 1 à 10 ml.

11. Utilisation de l'appareillage selon l'une des revendications 1 à 10 dans un procédé d'alignement de macromolécule(s) sur la surface S d'un support, dans lequel on fait se déplacer sur ladite surface S la ligne triple S/A/B (ménisque) résultant du contact d'un solvant A avec la surface S et un milieu B, lesdites macromolécules ayant une partie, notamment une extrémité, ancrée sur la surface S, l'autre partie, notamment l'autre extrémité, étant en solution dans le solvant A.

12. Utilisation d'un appareillage selon l'une des revendications 1 à 8 dans un procédé de mise en évidence, de séparation et/ou de dosage d'une macromolécule dans un échantillon, dans lequel on utilise un procédé d'alignement selon la revendication 11 dans lequel se trouve fixée sur la surface S une molécule à activité biologique capable de reconnaître ladite macromolécule de l'échantillon et en ce que la mise. en évidence, la séparation ou le dosage sont effectués grâce à un réactif fluorescent ou non détectant la présence de la molécule fixée ou ladite macromolécule.

13. Utilisation de l'appareillage selon l'une des revendications 1 à 10 dans un procédé de mise en évidence d'une macromolécule consistant en une séquence d'ADN ou d'une protéine dans un échantillon, selon la revendication 10, **caractérisée en ce que** :
- on met l'échantillon correspondant au solvant 1, dans lequel ladite macromolécule est en solution, en contact avec la surface du support dans des conditions de formation d'un hybride ADN/ADN,ADN/ARN ou de formation du produit de réaction protéine/protéine,
- l'hybride ou le produit de réaction étant marqué et ancré en une partie, le reste étant en solution, on l'étire par déplacement du ménisque créé par le contact du solvant avec la surface pour orienter les macromolécules et on effectue la mesure, l'observation des macromolécules ainsi orientées.

14. Utilisation d'un appareillage selon l'une des revendications 1 à 10 dans un procédé de cartographie physique d'un gène sur un ADN génomique dans lequel l'ADN est aligné et/ou mis en évidence selon l'une des revendications 11 à 13.

15. Utilisation d'un appareillage selon l'une des revendications 1 à 10 pour la mise en oeuvre d'un procédé de diagnostic d'une pathologie liée à la présence ou à l'absence d'une séquence d'ADN donnée spécifique de ladite pathologie.

16. Utilisation d'un appareillage selon l'une des revendications 1 à 10 pour la mise en oeuvre d'un procédé de préparation d'un gène dans lequel on identifie la position dudit gène sur l'ADN génomique aligné à l'aide d'une sonde spécifique dudit gène et on amplifie par PCR in situ la séquence dudit gène.

## Patentansprüche

1. Vorrichtung zur Parallel-Ausrichtung von Makromolekülen auf der Oberfläche S eines festen Trägers, **dadurch gekennzeichnet, daß** sie folgende Merkmale aufweist:
- einen Behälter (1), der dazu bestimmt ist, eine Lösung (2) aufzunehmen, die die auszurichtenden Makromoleküle enthält,
- einen festen Träger mit der Oberfläche S, die zur Befestigung der genannten Makromoleküle ausgebildet ist,
- Mittel zum Eintauchen des genannten Trägers (4) in die genannte Lösung (2), um einen Meniskus zu bilden, der durch die dreifache Übergangsfläche von Oberfläche S, Lösung und Luft erzeugt ist, und
- Mittel zum geradlinigen, relativen Versetzen der Oberfläche S und der Oberfläche der genannten Lösung, um die Einstellung einer parallelen Ausrichtung der Makromoleküle auf der Oberfläche zu gestatten.

2. Vorrichtung nach Anspruch 1, **dadurch gekennzeichnet, daß** die genannten Mittel zum Eintauchen des Trägers und die genannten Mittel zum relativen Versetzen des Meniskus und der Oberfläche S aus den selben Mitteln zur vertikalen Translation (3 bis 6) der genannten Oberfläche S senkrecht zur Oberfläche der genannten Lösung bestehen.

3. Vorrichtung nach Anspruch 1 oder 2, **dadurch gekennzeichnet, daß** die genannten Mittel zur vertikalen Translation der Oberfläche S die folgenden Merkmale aufweisen:
- Mittel (6) zum Greifen des festen Trägers, und
- Führungs- und Antriebsmittel (5), um die Translation der genannten Greifmittel längs einer vertikalen Achse (3) hervorzurufen, die senkrecht zum Behälter angeordnet ist.

4. Vorrichtung nach Anspruch 3, **dadurch gekennzeichnet, daß** die genannten Mittel zur vertikalen Translation der Oberfläche S die folgenden Merkmale aufweisen:
- Mittel zum Greifen des festen Trägers, die aus einer vertikalen Stange (3) bestehen, die senkrecht zum genannten Behälter angeordnet ist und Mittel (6) zum Festklemmen des festen Trägers aufweisen, und
- Führungs- und Antriebsmittel zum Steuern der vertikalen Translation der Stange zwischen einer unteren Eintauchlage und einer oberen, zurückgenommenen Lage.

5. Vorrichtung nach einem der Ansprüche 1 bis 4, **dadurch gekennzeichnet, daß** die Vorrichtung außerdem die folgenden Merkmale aufweist:
- ein erstes Lagerabteil für feste Träger, besonders für Plättchen, vor dem Eintauchen in den genannten Behälter, und
- ein zweites Lagerabteil für feste Träger, besonders für Plättchen, auf denen die Makromoleküle nach ihrem Zurückziehen aus dem genannten Behälter ausgerichtet sind.

6. Vorrichtung nach den Ansprüchen 4 und 5, **dadurch gekennzeichnet, daß** sie außerdem Mittel aufweist, um die genannten, festen Träger aufeinanderfolgend senkrecht zum ersten Abteil, zum genannten Behälter und zum genannten, zweiten Abteil darzubieten.

7. Vorrichtung nach den Ansprüchen 4 und 6, **dadurch gekennzeichnet, daß** die Vorrichtung, wenn die genannten Abteile und der genannte Behälter ausgerichtet sind, außerdem Führungs- und Antriebsmittel aufweist, um die horizontale Translation der Stange hervorzurufen, wenn sie sich in der oberen Lage befindet, um die genannten, festen Träger senkrecht zu den genannten Abteilen und zum genannten Behälter darzubieten.

8. Vorrichtung nach einem der Ansprüche 1 bis 7, **dadurch gekennzeichnet, daß** sie eine Vorrichtung zum Übewachen des pH-Wertes in der Lösung aufweist, die im genannten Behälter enthalten ist.

9. Vorrichtung nach einem der Ansprüche 1 bis 8, **dadurch gekennzeichnet, daß** die genannten Mittel zum Versetzen des Meniskus die Versetzung des Meniskus mit einer Geschwindigkeit von 10 bis 100 µm/s gestatten.

10. Vorrichtung nach einem der Ansprüche 1 bis 9, **dadurch gekennzeichnet, daß** das Volumen des genannten Behälters' 1 bis 10 ml beträgt.

11. Verwendung der Vorrichtung nach einem der Ansprüche 1 bis 10 in einem Verfahren zum Ausrichten eines oder mehrerer Makromoleküle auf der Oberfläche S eines Trägers, worin man auf der genannten Oberfläche S die dreifache Linie S/A/B (Meniskus) sich versetzen läßt, die sich aus der Berührung eines Lösungsmittels A mit der Oberfläche S und einer Umgebung B ergibt, wobei die genannten Makromoleküle mit einem Teil, besonders mit einem Ende, auf der Oberfläche S verankert sind, während der andere Teil, besonders das andere Ende, sich im Lösungsmittel A in Lösung befindet.

12. Verwendung einer Vorrichtung nach einem der Ansprüche 1 bis 8 in einem Verfahren zum Nachweis, zur Abtrennung und/oder zur Analyse eines Makromoleküls in einer Probe, worin man ein Verfahren zum Ausrichten nach Anspruch 11 verwendet, in dem ein Molekül mit biologischer Aktivität auf der Oberfläche S fixiert ist, das imstande ist, das genannte Makromolekül der Probe zu erkennen, und daß der Nachweis, die Abtrennung oder die Analyse dank eines fluoreszierenden oder nicht-fluoreszierenden Reagens bewirkt wird, das die Anwesenheit des fixierten Moleküls oder des genannten Makromoleküls nachweist.

13. Verwendung der Vorrichtung nach einem der Ansprüche 1 bis 10 in einem Verfahren zum Nachweis eines Makromoleküls, das aus einer DNS-Sequenz oder einem Protein in einer Probe besteht, nach Anspruch 10, **dadurch gekennzeichnet, daß**:
- man die Probe entsprechend dem Lösungsmittel 1, in dem sich das genannte Makromolekül in Lösung befindet, in Kontakt mit der Oberfläche des Trägers bringt, und zwar unter den Bedingungen der Bildung eines DNS/DNS-, DNS/RNS-Hybrids oder der Bildung des Reaktionsprodukts Protein/Protein,
- man während das Hybrid oder Reaktionsprodukt mit einem Teil markiert und verankert ist und sich der Rest in Lösung befindet, es durch Verlagerung des Meniskus, der durch den Kontakt des Lösungsmittels mit der Oberfläche erzeugt wird, streckt, um den Makromolekülen eine Orientierung zu verleihen, und man die Messung und die Beobachtung der so orientierten Makromoleküle bewirkt.

14. Verwendung einer Vorrichtung nach einem der Ansprüche 1 bis 10 in einem Verfahren zur physikalischen Kartographie eines Gens auf einer genomischen DNS, worin die DNS nach einem der Ansprüche 11 bis 13 ausgerichtet und/oder nachgewiesen wird.

15. Verwendung einer Vorrichtung nach einem der Ansprüche 1 bis 10 zur Durchführung eines Diagnoseverfahrens einer Pathologie, die mit der Anwesenheit oder Abwesenheit einer gegebenen, für die genannte Pathologie spezifischen DNS-Sequenz verbunden ist.

16. Verwendung einer Vorrichtung nach einem der Ansprüche 1 bis 10 für die Durchführung eines Verfahrens zur Herstellung eines Gens, in dem man die Position des genannten Gens auf der genomischen DNS identifiziert, die mit Hilfe einer für das genannte Gen spezifischen Sonde ausgerichtet ist, und man in situ die Sequenz des genannten Gens durch PCR amplifiziert.

## Claims

1. Apparatus for the parallel alignment of macromolecules on the surface S of a solid support, **characterized in that** it comprises:
- a container (1) intended to receive a solution (2) containing the macromolecules to be aligned,
- a solid support with a surface S configured so as to attach said macromolecules,
- means for immersing said support (4) in said solution (2) to form a meniscus created by the triple interface surface S/solution/air, and
- means for relative rectilinear movement of the surface S and of the surface of said solution to allow the formation of a parallel alignment of the macromolecules on the surface.

2. Apparatus according to Claim 1, **characterized in that** said means for immersing the support and said means for the relative movement of the meniscus and of the surface S consist of the same means for vertical translation (3 to 6) of said surface S perpendicularly to the surface of said solution.

3. Apparatus according to Claim 1 or 2, **characterized in that** said means for vertical translation of the surface S comprise:
- means (6) for gripping the solid support, and
- guiding and motor means (5) for causing translation of said gripping means along a vertical axis (3) placed perpendicularly to said container.

4. Apparatus according to Claim 3, **characterized in that** said means for vertical translation of the surface S comprise: i
- means for gripping the solid support consisting of a vertical rod (3) placed perpendicularly to said container and supporting means (6) for pinching the solid support, and
- guiding and motor means for controlling the vertical translation of the rod between a low immersion position and a high withdrawn position.

5. Apparatus according to one of Claims 1 to 4, **characterized in that** the apparatus comprises in addition:
- a first compartment for storing solid supports, especially slides, before immersion in said container, and
- a second compartment for storing solid supports, especially slides, on which the macromolecules are aligned after their withdrawal from said container.

6. Apparatus according to Claims 4 and 5, **characterized in that** it comprises, in addition, means for successively presenting said solid supports perpendicularly to said first compartment of said container and to said second compartment.

7. Apparatus according to Claims 4 and 6, **characterized in that** when said compartments and said container are aligned, the apparatus comprises, in addition, guiding and motor means to bring about the horizontal translation of the rod when it is in the raised position so as to allow said solid supports to be presented perpendicularly to said compartments and to said container.

8. Apparatus according to one of Claims 1 to 7, **characterized in that** it comprises a device for controlling the pH in the solution contained in said container.

9. Apparatus according to one of Claims 1 to 8, **characterized in that** said means for moving the meniscus allow the movement of the meniscus at a speed of 10 to 100 µm/sec.

10. Apparatus according to one of Claims 1 to 9, **characterized in that** the volume of said container is from 1 to 10 ml.

11. Use of the apparatus according to one of claims 1 to 10 in a process for aligning a macromolecule (macromolecules) on the surface S of a support, in which the triple line S/A/B (meniscus) resulting from the contact between a solvent A and the surface S and a medium B is caused to move on said surface S, said macromolecules having a part, especially an end, anchored on the surface S, the other part, especially the other end, being in solution in the solvent A.

12. Use of an apparatus according to one of Claims 1 to 8 in a process for detecting, separating and/or assaying a macromolecule in a sample, in which a process for alignment according to Claim 11 is used in which a molecule with biological activity capable of recognizing said sample macromolecule becomes attached to the surface S, and in that the detection, separation or assay are carried out using a reagent, fluorescent or otherwise, which detects the presence of the attached molecule or said macromolecule.

13. Use of the apparatus according to one of Claims 1 to 10 in a process for detecting a macromolecule consisting of a DNA sequence or a protein in a sample, according to Claim 10, **characterized in that**:
- the sample corresponding to solvent 1, in which said macromolecule is in solution, is brought into contact with the surface of the support under conditions for forming a DNA/DNA, DNA/RNA hybrid or for forming the protein/protein reaction product,
- the hybrid or the reaction product being labeled and anchored in one part, the remainder being in solution, it is stretched by the movement of the meniscus created by the contact between the solvent and the surface in order to orientate the macromolecules and the measurement or the observation of the macromolecules thus orientated is carried out.

14. Use of an apparatus according to one of Claims 1 to 10 in a process for the physical mapping of a gene on a genomic DNA in which the DNA is aligned and/or detected according to one of Claims 11 to 13.

15. Use of an apparatus according to one of Claims 1 to 10 for carrying out a process for the diagnosis of a pathology linked to the presence or absence of a given DNA sequence specific for said pathology.

16. Use of an apparatus according to one of Claims 1 to 10 for carrying out a process for preparing a gene in which the position of said gene on the aligned genomic DNA is identified with the aid of a probe specific for said gene and the sequence of said gene is amplified by in situ PCR.
